# EUROPEAN PATENT SPECIFICATION

(11) **EP 2 016 900 B1**
(45) Date of publication and mention of the grant of the patent: **11.07.2018**
(21) Application number: 07743125.2
(22) Date of filing: 10.05.2007
(51) Int. Cl.: A61B 5/151

(54) **PIERCING INSTRUMENT AND PIERCING NEEDLE CARTRIDGE**
STECHINSTRUMENT UND STECHNADEL-KASSETTE
INSTRUMENT DE PERCÉE ET CARTOUCHE D'AIGUILLE À PERFORER

(30) Priority: 10.05.2006 JP 2006131357
(43) Date of publication of application: 21.01.2009
(73) Proprietor: Panasonic Healthcare Holdings Co., Ltd., Tokyo 105-8433 (JP)
(72) Inventor: NAGAO, Akio, Osaka 540-6207 (JP); TAKANO, Michiyasu, Osaka 540-6207 (JP)
(74) Representative: Grünecker Patent- und Rechtsanwälte PartG mbB
(86) International application number: PCT/JP2007/059690
(87) International publication number: WO 2007/129757

(56) References cited:
- EP-A- 1 561 421
- WO-A-03/013357
- WO-A-2006/046570
- WO-A1-2006/046570
- JP-A- 2006 305 143
- US-A1- 2005 038 465
- US-A1- 2005 143 771
- US-A1- 2005 143 771
- US-A1- 2006 052 809

## Description

### Technical Field

The present invention relates to a puncture device and puncture needle cartridge. More particularly, the present invention relates to a puncture device and puncture needle cartridge for collecting blood.

### Background Art

Conventionally, various puncture devices for collecting blood and disposable lancets used for the puncture devices, have been developed. When a lancet is attached to or removed from such a puncture device, there is a danger that the puncture needle exposed from one end of the lancet punctures the hand by error. To solve such a problem, a puncture needle cartridge with a cap that covers the puncture needle of a lancet is developed. Since there is no danger that the hand directly touches the puncture needle of the puncture needle cartridge, it is possible to attach the puncture device to the lancet safely (for example, see Patent Documents 1, 2 and 3) . WO 2006/046570 A1 and US 2005/0143771 A1 disclose a lancet with a puncture needle protected by a removable protection cap.
Patent Document 1: Japanese Patent Application Laid-Open No.2005-312763
Patent Document 2: International Publication No.2003/005907 Pamphlet
Patent Document 3: Japanese Patent Application Laid-Open No.HEI7-16218

### Disclosure of Invention

### Problems to be Solved by the Invention

The conventional puncture needle cartridge has a puncture needle at one end, a lancet body with a grip part to be held by a puncture device at the other end, and a protection cap provided separably from the lancet body. The puncture needle cartridge is used by being attached to the puncture device every time when puncturing is performed. By wrenching off the protection cap of the puncture needle cartridge attached to the puncture device, the protection cap is torn from the lancet body.

However, with the conventional puncture needle cartridge, cases may occur where the protection cap is tried to be removed from the lancet body while the protection cap is not yet torn from the lancet body. When the protection cap is tried to be removed from the lancet body while the protection cap is not yet torn from the lancet body, an adequate hold between the grip part of the lancet body and the plunger of the puncture device holding the grip part, may be lost. When the adequate hold between the grip part and the plunger is lost, the depth of puncturing upon puncturing becomes inadequate or the tip of the puncture needle of the lancet is exposed from the puncture device. There is a danger that the exposed puncture needle may cause a needle-stick accident.

Upon the protection cap is removed from the lancet body, the present invention makes it possible to remove the protection cap from the lancet body of the puncture needle cartridge attached to the puncture device in a reliable manner, while maintaining an adequate hold of the lancet body by the puncture device (plunger) . Further, upon hold of the lancet body is lost by error operation, the present invention makes the protection cap unable to be removed from the lancet body.

An object of the present invention is to provide a safe puncture needle cartridge for preventing the tip of the puncture needle from sticking out from the puncture device and causing a needle-stick accident.

### Means for Solving the Problem

The first aspect of the present invention relates to a puncture needle cartridge according to claim 1.

A second aspect of the present invention relates to a puncture device according to claim 8.

### Advantageous Effect of the Invention

According to the puncture device or puncture needle cartridge of the present invention, if the protection cap is tried to be removed while the protection cap is not rotated or is not rotated enough and the protection cap is not torn from the lancet body, the protection cap cannot be removed from the lancet body, so that the whole of the lancet is removed from the puncture device body. Therefore, the protection cap cannot be removed from the lancet body when the lancet body is not held by the puncture device body firmly, so that there is no danger that the puncture needle is exposed and causes a needle-stick accident.

### Brief Description of Drawings

FIG.1 is a perspective view of the puncture device;
FIG.2 is a transparent view of the part to which the puncture needle cartridge is attached, in the first puncture device;
FIG.3 is a perspective view of puncture needle cartridge 102a;
FIG.4A is a perspective view of lancet 204a, FIG.4B is an enlarged view near claw 303a formed in the protection cap in the lancet, and FIG.4C is a plan view of the lancet seen from the upper side of the lancet body;
FIG.5A and FIG.5B are perspective views of puncture needle holder 201a seen from different angles;
FIG.6A shows a state where lancet 204a engages with puncture needle holder 201a, and FIG.6B shows a state where puncture needle cartridge 102a is formed while lancet 204a is engaged with puncture needle holder 201a and the right part in the figure shows a cross section;
FIG.7 shows how the protection cap is removed from the puncture needle holder of the puncture needle cartridge, where FIG.7A shows an initial state where the lancet is attached to the puncture needle holder, FIG.7B shows a state where the protection cap is rotated predetermined degrees, and FIG.7C shows a state where the claw is guided by the slope and moves so that the protection cap is removed;
FIG. 8A shows a state where the claw of the protection cap contacts with the slope of the puncture needle holder and the contacting face between the claw and the slope has the sloping shape, FIG.8B shows a state where the claw of the protection cap contacts with the slope of the puncture needle holder and the contacting face between the claw and the slope has the shape of a semicircle, and FIG.8C shows a state where the claw of the protection cap contacts with the slope of the puncture needle holder and the contacting face between the claw and the slope has the shape of a step;
FIG.9 is an development view showing a state where protection cap 202a is inversely rotated in puncture needle cartridge 102a, where FIG.9A shows a state where claw 303a of the protection cap is latched in inverse rotation preventing rib 403a, FIG.9B shows a state where the protection cap is rotated beyond the inverse rotation preventing rib, and FIG.9C shows a state where the claw moves through a cutout part and the protection cap is removed;
FIG.10A is a perspective view of lancet 204b, FIG.10B is an enlarged view near claw 303b formed in the protection cap of the lancet, and FIG.10C is a plan view of the lancet seen from the upper side of the lancet body;
FIG.11A and FIG.11B are perspective views of puncture needle holder 201b seen from different angles;
FIG.12 shows the relationship between claw 303b of the protection cap and flange 401b and guide 402b of the puncture needle holder, where the claw of the protection cap is in plane contact with the guide of the puncturing holder;
FIG.13 shows the relationship between the claw of the protection cap and the flange and guide of the puncture needle holder, where the claw of the protection cap is in line contact with the guide of the puncture needle holder;
FIG.14 shows the relationship between the claw of the protection cap and the flange and guide of the puncture needle holder, where the claw of the protection cap is in line contact with the guide of the puncture needle holder;
FIG.15A is a transparent perspective view of the part to which lancet 204c is attached in one example of second puncture device 101c;
FIG.15B is a perspective view of lancet 204c of the puncture device shown in FIG.15A;
FIG.15C is a perspective view of puncture device body 101c of the puncture device shown in FIG.15A;
FIG.16A is a transparent perspective view of the part to which lancet 204d is attached in another example of second puncture device 101d;
FIG.16B is a perspective view of lancet 204d of the puncture device shown in FIG.16A; and
FIG.16C is a perspective view of plunger 103d of the puncture device shown in FIG.16A.

### Best Mode for Carrying Out the Invention

Both the puncture needle cartridge and puncture device of the present invention have a lancet that includes a protection cap and lancet body. That is, the puncture needle cartridge has a lancet and a puncture needle holder that holds the lancet movably, and the puncture needle holder of the puncture needle cartridge is attached to the puncture device (first puncture device) . On the other hand, a puncture device (second puncture device) has a lancet not held by the puncture needle holder and a holding part that holds the lancet movably.

### The puncture device

As described above, there are cases where the puncture device has a lancet, puncture device body and puncture needle holder (first puncture device), and cases where the puncture device does not have a puncture needle holder (second puncture device).

### The first puncture device

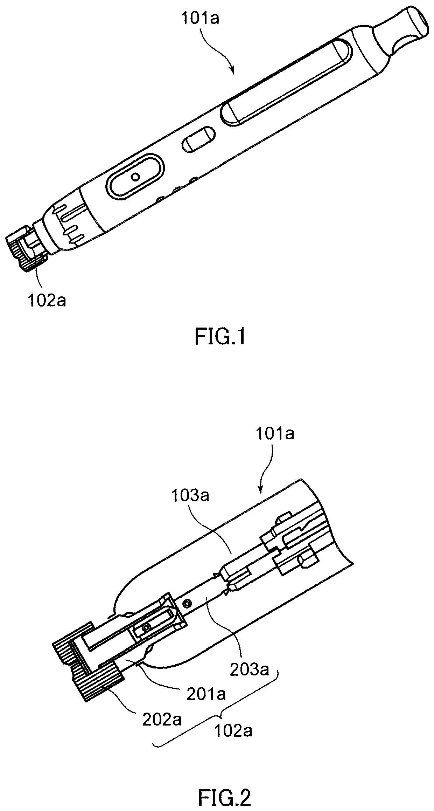

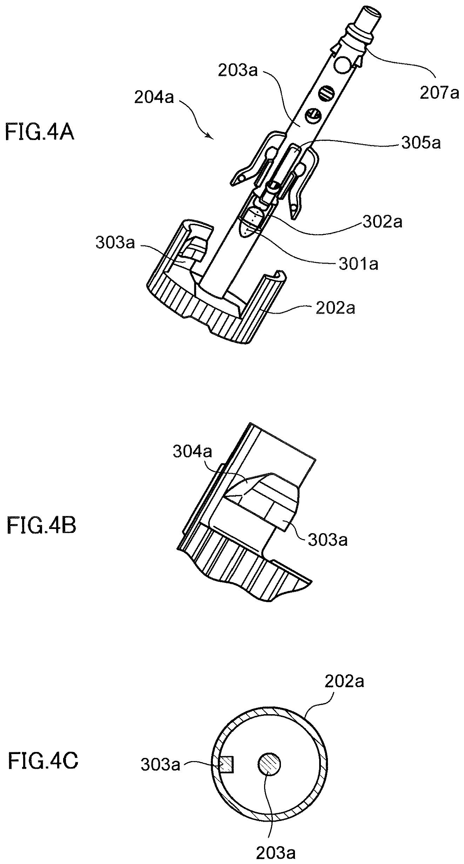

FIG.1 is a perspective view of an example of the first puncture device. Puncture needle cartridge 102a is attached to puncture device body 101a. FIG.2 is a transparent view of the area where puncture needle cartridge 102a is attached to puncture device body 101a in an example of the first puncture device (puncture device having a puncture needle holder). Puncture needle cartridge 102a has lancet 204a (see FIG.4) including protection cap 202a and lancet body 203a, and puncture needle holder 201a. One end of lancet body 203a of puncture needle cartridge 102a is held by plunger 103a of puncture device body 101a.

As shown in FIG.2, the lancet included in the first puncture device is integrated with puncture needle holder 201a so as to become one part of the puncture needle cartridge. The puncture needle holder is attached to the puncture device body, and the lancet is attached to the puncture device body via the puncture needle holder.

### The second puncture device

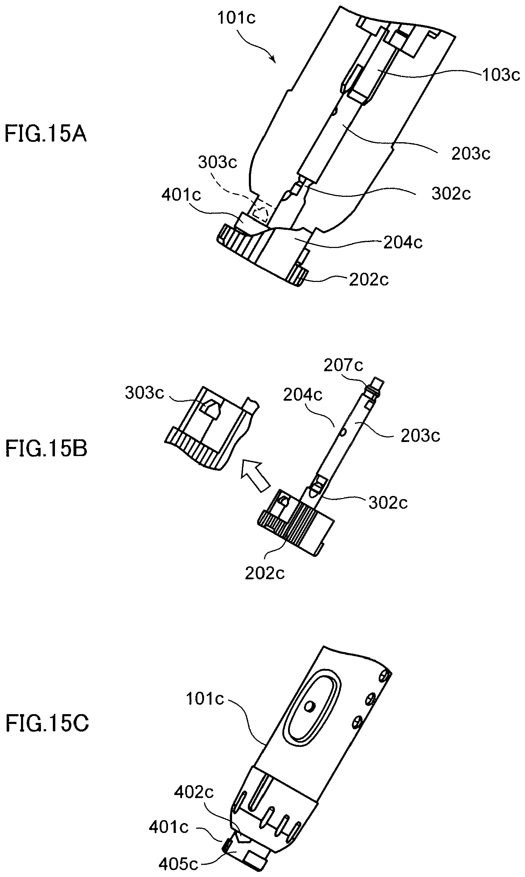

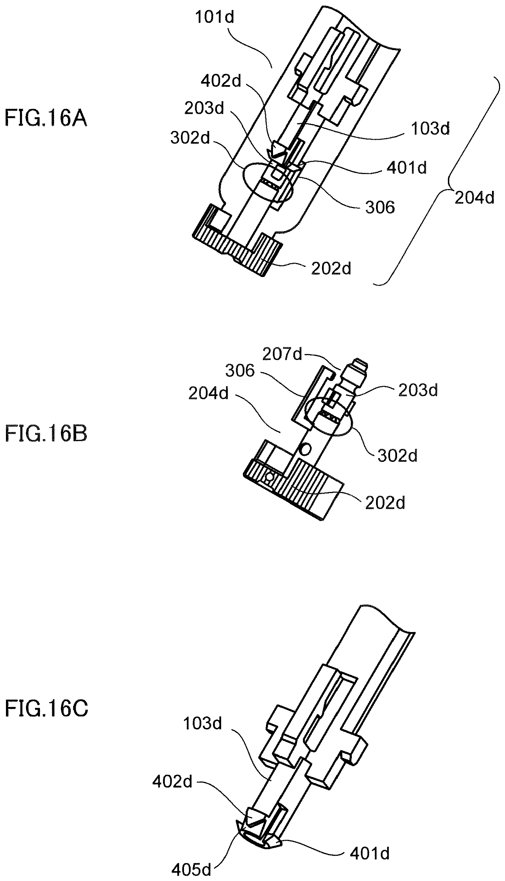

On the other hand, FIG.15A and FIG.16A are transparent views showing the areas where the lancets (204c and 204d) are attached to the puncture device bodies (101c and 101d) in an example of the second puncture device (puncture device not having a puncture needle holder) . One end of the lancet bodies (203c and 203d) constituting the lancet is held by the plungers (103c and 103d) of the puncture device body.

Further, claw 303c of the protection caps (202c and 202d) or angle guide 306 is engaged with puncture device body 101c. These are described in detail later.

### The puncture needle cartridge

The puncture needle cartridge has a lancet and a puncture needle holder that holds the lancet movably. The lancet has a lancet body with a puncture needle, and a protection cap that covers and protects the puncture needle, and the lancet body and the protection cap are connected separably via a connecting part. By rotating the protection cap with respect to the axis of the lancet body, the connecting part is cut so that the protection cap and the lancet body are disconnected and the protection cap can be removed from the lancet body.

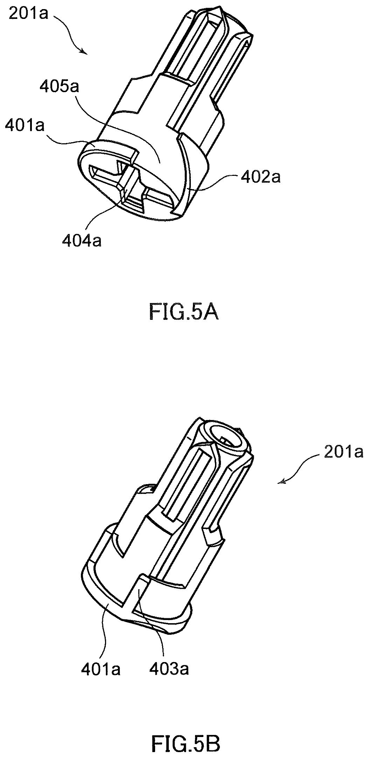

The first convex part or concave part is formed in the protection cap (see 303a in FIG.4). On the other hand, the second convex part or concave part is formed in the puncture needle holder (see 401a in FIG.5) . When the puncture needle holder is attached to the lancet, the first convex part or concave part and the second convex part or concave part are engaged. By this engagement, the protection cap cannot move in the direction of the axis of the lancet body and cannot be removed from the puncture needle holder.

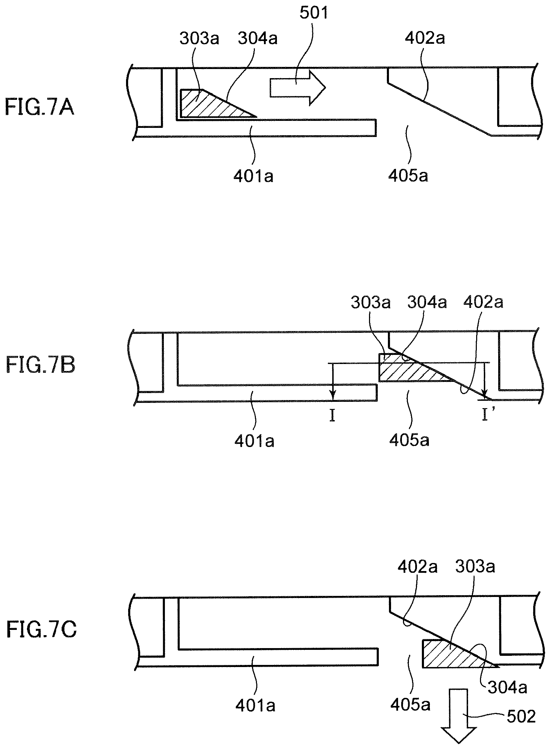

The puncture needle holder also has a cutout part (see 405a in FIG. 5A) where the second convex part or concave part is not formed, in addition to the second convex part or concave part formed in the puncture needle holder. Further, a slope or guide part (see 402a in FIG.5A) is formed near the cutout part. If the protection cap of the lancet attached to the puncture needle holder is rotated predetermined degrees, the first convex part or concave part moves to the cutout part, so that the first convex part or concave part and the second convex part or concave part are disengaged. That is, by rotating the protection cap predetermined degrees, the first convex part or concave part contacts with the guide part, and, by further rotating the protection cap, the first convex part or concave part is guided by the guide part and pass through the cutout part, so that the protection cap is removed from the puncture needle holder (see FIG.7).

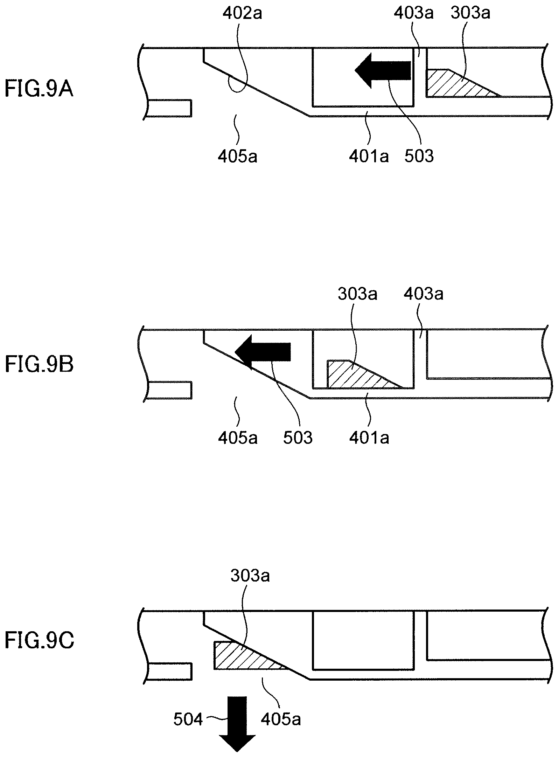

The protection cap of the lancet attached to the puncture needle holder may be rotatable in both directions with respect to the axis of the lancet body or may be rotatable in only one direction. To make the protection cap rotatable in only one direction, a restriction part (see 403a in FIG.5B) restricting the rotation direction may be provided in the second convex part or concave part of the puncture needle holder. Further, to make the protection cap rotatable in only one direction a third convex part or concave part (see 401a in FIG.9) may be provided in the puncture needle holder. The third convex part or concave part engages with the first convex part or concave part of the protection cap if the protection cap rotates in the inverse direction against the restriction of the restriction part.

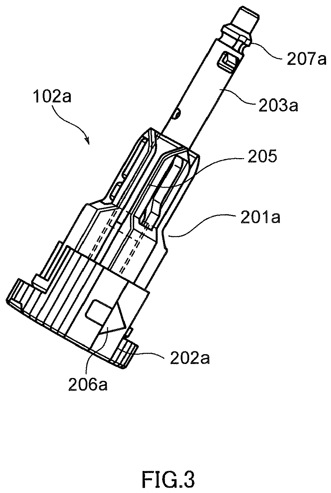

FIG.3 is a perspective view of puncture needle cartridge 102a included in the first puncture device shown in FIG.2. Puncture needle cartridge 102a has lancet 204a and puncture needle holder 201a. Lancet 204a (see FIG.4) has lancet body 203a and protection cap 202a. Grip part 207a is formedat one end of lancet body 203a, and a puncture needle (not shown) is provided at the other end. Protection cap 202a covers and protects the puncture needle. Further, arrow 206a, which is a sign to show the direction to rotate protection cap 202a to tear protection cap 202a from lancet body 203a, may be given to protection cap 202a. Upon attaching puncture needle cartridge 102a to puncture device body 101a, grip part 207a is held by the tip part of plunger 103a (see FIG.2, described later) of puncture device body 101a.

FIG.4A is a perspective view of lancet 204a. Lancet 204a shown in FIG.4A has lancet body 203a with puncture needle 301a and protection cap 202a that covers puncture needle 301a. Lancet body 203a and protection cap 202a are formed with an elastic material such as resin, for example, polyethylene resin, in an integrated manner, and are separably connected via connecting part 302a.

Protection cap 202a can rotate with respect to the axis of lancet body 203a, and, when protection cap 202a rotates predetermined degrees, the connecting part 302a is preferably cut so that protection cap 202a and lancet body 203a are disconnected. That is, "separable" means that, when the protection cap is rotated predetermined degrees with respect to the axis of the lancet body, the connection between the protection cap and the lancet body is broken or the protection cap and the lancet body are disengaged each other and the protection cap can be removed.

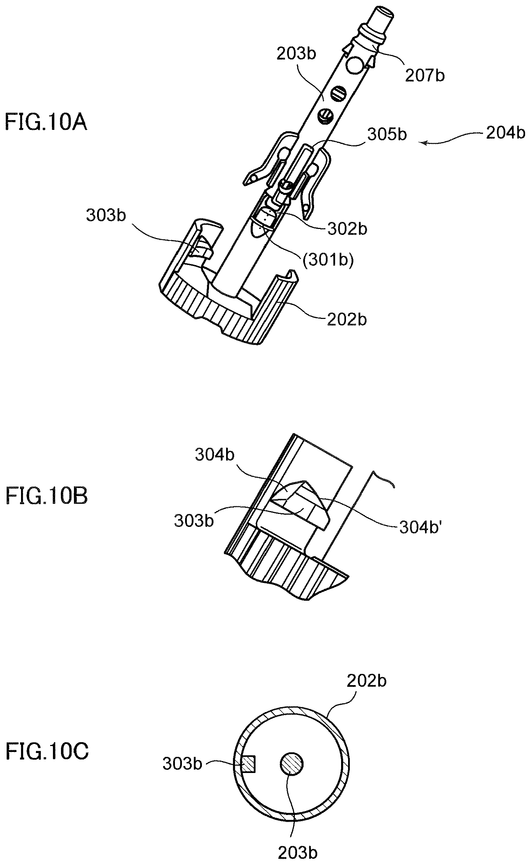

Cross-shaped rib 305a is formed in lancet body 203a. Further, as shown in FIG.4B (enlarged view near claw 303a) and FIG.4C (plan view of the lancet seen from grip part 207a of the lancet body), the interior of protection cap 202a is hollow, and claw 303a is provided in the inner periphery wall of protection cap 202a. Further, as shown in FIG.4B, inclination 304a is formed in the side of claw 303a. Claw 303a of lancet 204a shown in FIG. 4B may be inclined in one side. On the other hand, claw 303b of lancet 204b shown in FIG.10B is inclined in two sides. Lancet 204b will be described in detail later. Inclination 304a of claw 303a contacts with slope 402a (see FIG.5A) described later.

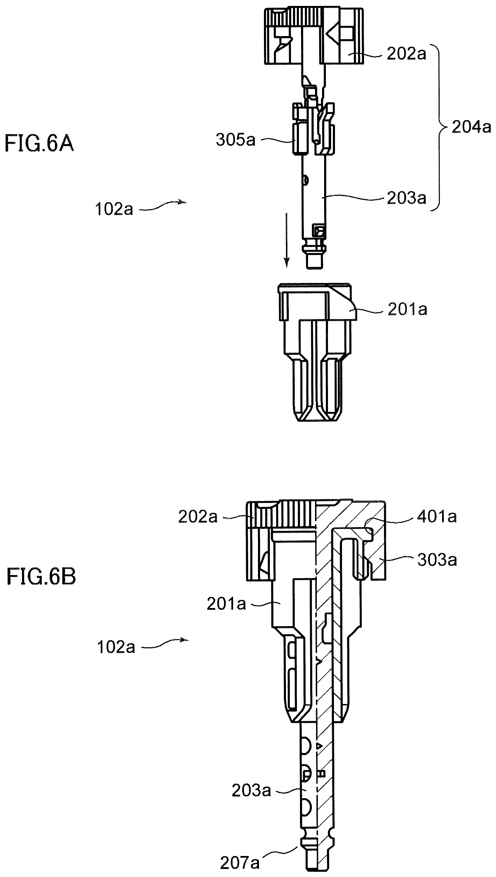

FIG.5A and FIG.5B are perspective views of puncture needle holder 201a, seen from different directions. Cross-shaped groove 404a is provided at puncture needle holder 201a. By engaging cross-shaped rib 305a (see FIG.4A) provided at lancet body 203a with cross-shaped groove 404a, lancet body 203a and puncture needle holder 201a are integrated (see FIG.6). By engaging cross-shaped rib 305a with cross-shaped groove 404a, the relative position of puncture needle holder 201a and protection cap 202a in the initial state with respect to the axis of lancet body 203a, is defined.

Flange 401a is formed in the outer face (outer periphery face of the cylinder) of puncture needle holder 201a, and claw 303a (see FIG.4) of protection cap 202a is engaged with flange 401a. Cutout part 405a is provided so that claw 303a of protection cap 202a and flange 401a are disengaged when protection cap 202a rotates predetermined degrees with respect to the axis of lancet body 203a. Further, slope 402a is formed in the outer face of puncture needle holder 201a so that, when protection cap 202a rotates predetermined degrees with respect to the axis of lancet body 203a, inclination 304a of claw 303a contacts with slope 402a.

Further, restriction part 403a restricting the rotation direction of protection cap 202a is formed in the outer face (outer periphery face of the cylinder) of puncture needle holder 201a. Restriction part 403a will be described in detail later.

As shown in FIG.6, puncture needle cartridge 102a is assembled by engaging lancet 204a with puncture needle holder 201a. That is, puncture needle cartridge 102a is assembled by inserting cross-shaped rib 305a of lancet 204a in a predetermined position where cross-shaped rib 305a engages with cross-shaped groove 404a of puncture needle holder 201a. Puncture needle cartridge 102a is assembled based on the positional relationship between cross-shaped groove 404a and cross-shaped rib 305a (such that cross-shaped rib 305a engages with cross-shaped groove 404a) and the relationship between claw 303a and cutout part 405a in their initial positions immediately after puncture needle cartridge 102a is assembled (such that claw 303a is not placed in the position of cutout part 405a).

FIG.6B is a partial cross-sectional view (only the right part shows a cross-section) of puncture needle cartridge 102a in the initial position. Upon the assembling, claw 303a of protection cap 202a is engaged with flange 401a of puncture needle holder 201a. The engagement of claw 303a and flange 401a makes it impossible to pull out protection cap 202a from lancet body 203a in the axial direction (in the vertical directions in FIG.6B). Therefore, if protection cap 202a is pulled for the purpose of removing protection cap 202a from lancet body 203a without rotating protection cap 202a of puncture needle cartridge 102a attached to puncture device body 101a with respect to the axis of lancet body 203a, the whole of puncture needle cartridge 102a has to be removed from puncture device 101a. Therefore, even if protection cap 202a is pulled by force, an adequate hold of lancet body 203a by the puncture device (plunger) is not lost, the depth of puncturing does not change, or the tip of the needle of the lancet does not protrude from the puncture device, so that it is possible to prevent a needle-stick accident by the protruding needle.

Although, in puncture needle cartridge 102a shown in FIG.6A and FIG.6B, claw 303a provided in protection cap 202a, and flange 401a provided in puncture needle holder 201a, are engaged with each other, it is only necessary to prevent protection cap 202a from being pulled out from puncture needle holder 201a, and therefore any engaging method is possible. For example, a concave part, for example, in the shape of a groove, may be formed in puncture needle holder 201a.

FIG.7A to FIG.7C show how protection cap 202a is removed from lancet 204a of puncture needle cartridge 102a. FIG.7A to FIG.7C simply illustrate the relationships between flange 401a, cutout part 405a and slope 402a of puncture needle holder 201a and claw 303a of protection cap 202a, with development plan view of the cylindrical shape of the side of the holder.

FIG.7A shows the initial state of puncture needle cartridge 102a (the state where lancet 204a is attached to puncture needle holder 201a, see FIG.6B). By rotating protection cap 202a in a predetermined direction (direction of arrow 501) from the state of FIG.7A, claw 303a moves to the right in the figure, and inclination 304a (see FIG.4B) of the side of claw 303a contacts with slope 402a of puncture needle holder 201a (see FIG.7B). As described above, the direction (direction of arrow 501) of rotation of protection cap 202a may be provided in the protection cap (see FIG.3), and the protection cap is prevented from rotating in a direction other than the predetermined direction.

The connecting part 302a (see FIG.4) is preferably cut so that protection cap 202a and lancet body 203a are disconnected when protection cap 202a rotates until inclination 304a of the side of claw 303a contacts with slope 402a of puncture needle holder 201a as shown in FIG.7B. Protection cap 202a preferably rotates 90 to 270 degrees, more preferably, 150 to 180 degrees from the initial state of FIG.7A to the state of FIG.7B.

Further, in the state shown in FIG.7B, claw 303a engaged with flange 401a of puncture needle holder 201a moves to a position corresponding to cutout part 405a where flange 401a is lacking.

From the state of FIG.7B, when protection cap 202a is further rotated in the direction of arrow 501, claw 303a moves along slope 402a of puncture needle holder 201a, so that only protection cap 202a in lancet 204a is removed from lancet 204a in the direction of arrow 502 (see FIG.7C).

In the state of FIG.7B, if claw 303a of protection cap 202a does not contact with slope 402a of puncture needle holder 201a adequately, claw 303a may not be guided by slope 402a, and protection cap 202a may not be led in the direction of arrow 502 adequately. That is, cases may occur where inclination 304a and slope 402a are disengaged, and thereby claw 303a climbs over slope 402a in the direction of arrow 501. Therefore, even if slope 402a tries to guide claw 303a in the direction of arrow 502 shown in FIG.7C, slope 402a cannot guide claw 303a adequately. Therefore, it is preferable to form the shapes of claw 303a and slope 402a adequately so as to prevent claw 303a and slope 402a from being disengaged.

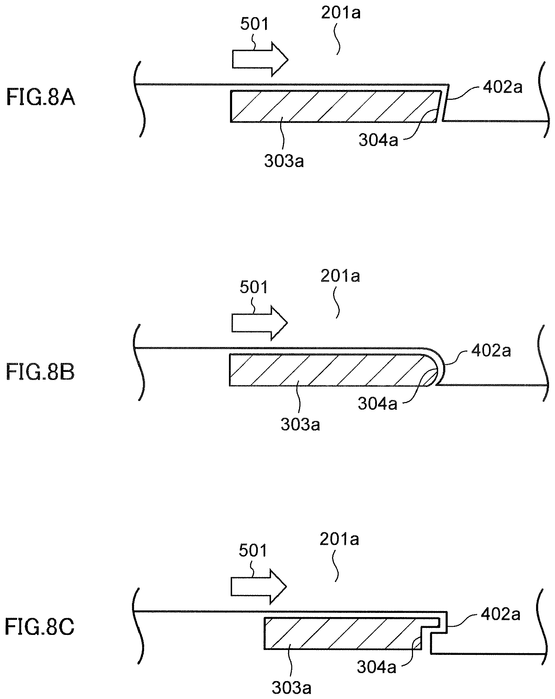

FIG.8A to FIG. 8C are cross-sectional views of I-I' of FIG.7B, and show examples of the structure for preventing claw 303a of protection cap 202a and slope 402a of puncture needle holder 201a from easily being disengaged. For example, as shown in FIG.8A, when inclination 304a of claw 303a contacts with slope 402a, the inner periphery side of claw 303a may engage with slope 402a more deeply than the outer periphery side of claw 303a. Alternatively, as shown in FIG.8B, the contacting faces between inclination 304a and slope 402a may be a semicircle, or, as shown in FIG.8C, the contacting faces between inclination 304a and slope 402a may have the shape of a step.

By adopting the structures shown in FIG.8A to FIG.8C, even if the force of rotating protection cap 202a is excessive, slope 402a can control claw 303a adequately and keep claw 303a upward in FIG.8A to FIG.8C.

Similarly with FIG.7, FIG.9A to FIG.9C illustrate, the relationship between flange 401a of puncture needle holder 201a and claw 303a of protection cap 202a in puncture needle cartridge 102a in a simple manner with development plan views of the shape of the side (cylindrical shape) of the holder. FIG.9 illustrates a case where protection cap 202a is rotated not in a predetermined direction (arrow 501 in FIG.7), but in the inverse direction of the predetermined direction (direction of arrow 503).

As shown in FIG.9A, inverse rotation preventing rib 403a is provided in puncture needle holder 201a (see FIG.5B). When protection cap 202a is rotated not in the predetermined direction (arrow 501 in FIG.7), but in the direction of arrow 503, inverse rotation preventing rib 403a engages with claw 303a of protection cap 202a, so that its rotation direction is restricted (FIG.9A).

However, when protection cap 202a is rotated further in the direction of arrow with an excessive force, a case may occur where claw 303a climbs over inverse rotation preventing rib 403a and protection cap 202a rotates. Particularly, when protection cap 202a is formed with a deformable elastic material, claw 303a of protection cap 202a is easy to climb over inverse rotation preventing rib 403a.

Even if claw 303a climbs over inverse rotation preventing rib 403a and protection cap 202a rotates until the state of FIG.9B, connecting part 202a connecting protection cap 202a and lancet body 203a is not cut. If protection cap 202a is pulled in the axial direction in this state, as described above, lancet body 203a is pulled out together with protection cap 202a, and therefore adequate engagement of lancet body 203a and the puncture device may be lost, which may cause a needle-stick accident.

Therefore, as shown in FIG.9B, flange 401a (matching the third convex part or concave part) is preferably provided at the side of arrow 503 from inverse rotation preventing rib 403a so that, even if claw 303a of protection cap 202a climbs over inverse rotation preventing rib 403a, protection cap 202a and puncture needle holder 201a can not be separated. By this means, in the position of FIG.9B, adequate engagement of lancet body 203a and the puncture device may not be lost by pulling out protection cap 202a in the axial direction, which makes the puncture device safe.

If protection cap 202a is further rotated in the direction of arrow 503 from the state shown in FIG.9B until the position in which connecting part 302a of lancet 204a is cut, as shown in FIG.9C, claw 303a passes through cutout part 405a of flange 401a of puncture needle holder 201a, so that protection cap 202a is removed from lancet 204a. The rotation angle of protection cap 202a from the state of FIG.9A to the state of FIG.9C is preferably 90 degrees to 270 degrees, and, more preferably, 150 degrees to 180 degrees.

Claw 303a of protection cap 202a is preferably configured so as to prevent Claw 303a from easily climbing over inverse rotation preventing rib 403a by adjusting the contacting face between inverse rotation preventing rib 403a of puncture needle holder 201a and claw 303a of protection cap 202a in the same way as the contacting face between inclination 304a of claw 303a and slope 402a (see FIG.8A to FIG.8C).

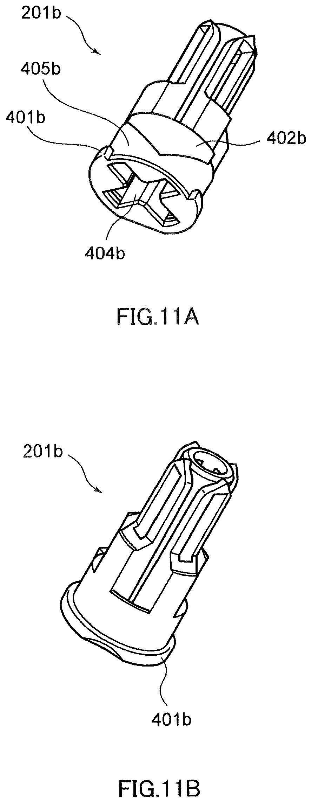

As shown in FIG.9A to FIG.9C, the direction of rotation of protection cap 202a may be restricted to a predetermined direction by providing inverse rotation preventing rib 403a in puncture needle holder 201a, but the direction of rotation does not always have to be restricted, and protection cap 202a may be rotated in both rotation directions. FIG. 10A to FIG.10C and FIG.11A to FIG.11B show lancet 204b and puncture needle holder 201b wherein protection cap 202b can rotate in both rotation directions.

Lancet 204b shown in FIG.10A to FIG.10C is the same as lancet 204a shown in FIG.4A to FIG.4C except for the shape of claw 303b. That is, lancet 204b has lancet body 203b and protection cap 202b which are connected separably via connecting part 302b. When protection cap 202b is rotated predetermined degrees with respect to the axis of lancet body 203b, connecting part 302b is cut so that protection cap 202b and lancet body 203b are disconnected, and protection cap 202b may be removed from lancet body 203b.

Grip part 207b is formed in one end of lancet body 203b, grip part 208b is held in the tip part of plunger 103a of the puncture device body (see FIG.2), puncture needle 301b is provided in the other end, and puncture needle 301b (not shown) is covered with protection cap 202b and protected. Further, cross-shaped rib 305b is formed in lancet body 203b.

As shown in FIG.10B and FIG.10C, the interior of protection cap 202b is hollow, and claw 303b is provided in the inner periphery wall of protection cap 202b. Further, inclinations 304b and 304b' are formed in the two sides of claw 303b of protection cap 202b shown in FIG.10A and FIG.10B.

Further, puncture needle holder 201b shown in FIG.11A to FIG.11B is the same as puncture needle holder 201a shown in FIG.5A and FIG.5B except that guide 402b is formed instead of slope 402a and a member corresponding to inverse rotation preventing rib 403a is not provided.

That is, flange 401b is formed in puncture needle holder 201b, and claw 303b of lancet 204b is engaged with flange 401b. This engagement restricts the movement of protection cap 202b in the axial direction of lancet body 203b. Further, cutout part 405b is provided in puncture needle holder 201b, and therefore, when protection cap 202b is rotated with respect to the axis of lancet body 203b, claw 303b guided by guide 402b passes through cutout part 405b, so that protection cap 202b is removed from lancet body 203b.

Guide 402b of puncture needle holder 201b is not particularly limited, but preferably has a symmetric shape, for example, a shape of "V." By forming guide 402b in a symmetric shape, when protection cap 202b of lancet 204b attached to puncture needle holder 201b is rotated predetermined degrees in one of rotation directions with respect to the axial direction of lancet body 203b, one of inclinations 304b and 304b' of claw 303b contacts with guide 402b. Claw 303b is guided by guide 402b and passes through cutout part 405b, so that protection cap 202b is removed from lancet body 203b by guide 402b of puncture needle holder 201b. In this way, protection cap 202b can be removed when protection cap 202b is rotated in one of rotation directions with respect to the axis of lancet body 203b, and a member corresponding to inverse rotation preventing rib 403b is not provided in puncture needle holder 201b (see FIG.11B).

Cross-shaped groove 404b is formed at puncture needle holder 201b, and cross-shaped rib 305b of lancet 204b engages with cross-shaped groove 404b, and the relative position of protection cap 202b and puncture needle holder 201b in the initial state is defined (in the same way as FIG.6B). In the initial state, flange 401b of puncture needle holder 201b and claw 303b of protection cap 202b are engaged. When protection cap 202b is rotated predetermined degrees with respect to the axis of lancet body 203b from the initial state, flange 401b and claw 303b of protection cap 202b are disengaged, and protection cap 202b is removed through cutout part 405b.

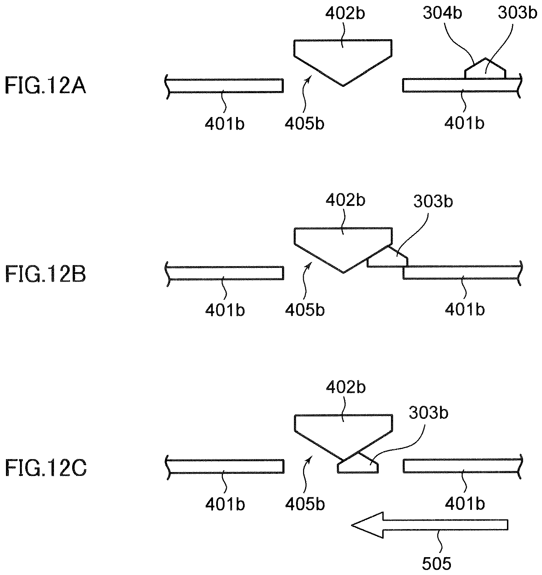

FIG.12 to FIG.14 illustrate how, in the puncture needle cartridge consisting of lancet 204b (see FIG.10A to FIG.10C) and puncture needle holder 201b (see FIG.11A and FIG.11B), claw 303b of protection cap 202b is engaged with flange 401b of puncture needle holder 201b and disengaged through cutout part 405b. Each of FIG.12 to FIG.14 illustrates development plan view of the cylindrical shape of the side of puncture needle holder 201b of the puncture needle cartridge in a simple manner.

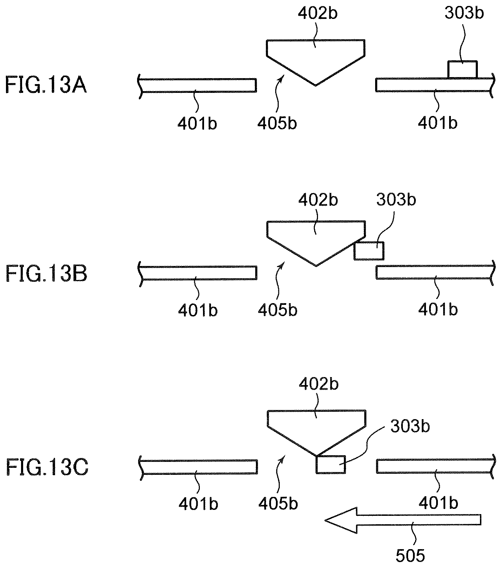

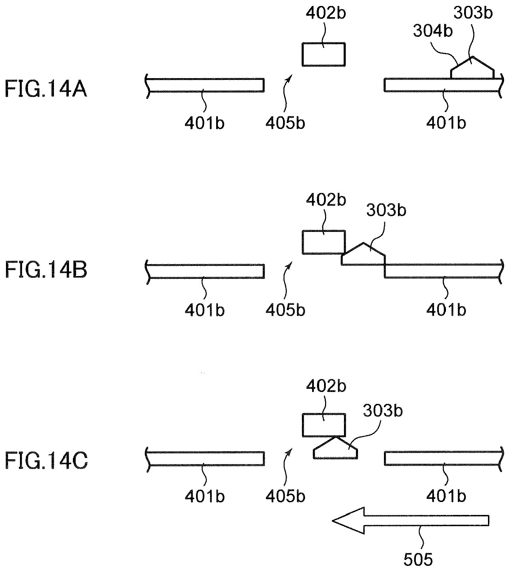

The puncture needle cartridges shown in FIG.12 to FIG.14 are different from each other in the shape of claw 303b of protection cap 202b or in the shape of guide part 402b of puncture needle holder 201b. That is, in the puncture needle cartridge shown in FIG.12, both of claw 303b and guide part 402b have sloping surfaces which plane-contact with each other. On the other hand, in the puncture needle cartridge shown in FIG.13 and FIG.14, claw 303b or guide part 402b has sharp edge which line-contacts with sloping surface of claw 303b or guide part 402b.

FIG.12A, FIG. 13A and FIG. 14A show an intermediate state between a state where protection cap 202b is rotated from the initial state of the puncture needle cartridge and a state where claw 303b is in contact with guide part 402b. Although protection cap 202b may be rotated in the direction of arrow 505 shown in the figures, or in the inverse direction, a case will be described where protection cap 202b is rotated in the direction of the arrow.

FIG.12B, FIG.13B and FIG.14B show a state where protection cap 202b is further rotated in the direction of arrow 505 and claw 303b contacts with guide part 402b. FIG.12C, FIG.13C and FIG.14C show a state where protection cap 202b is further rotated in the direction of arrow 505 from the state in FIG.12B, FIG.13B and FIG.14B. Guide part 402b and claw 303b interfere with each other, claw 303b moves along the inclination of guide part 402b or along the inclination of claw 303b (moves downward in the figures), and protection cap 202b is removed.

Next, FIG.15 and FIG.16 show an example of the second puncture device (puncture device not having a puncture needle holder). The puncture device does not have a puncture needle holder, so that the lancet is directly attached to the puncture device body. The lancet has a lancet body and a protection cap, and the lancet body and protection cap are connected separably via the connecting part. The grip part to be held by the plunger of the puncture device body is formed at one end of the lancet body and a puncture needle is formed at the other end. The protection cap covers and protects the puncture needle. When the protection cap is rotated with respect to the axis of the lancet body, the connecting part is cut so that the protection cap and the lancet body are disconnected and the protection cap can be removed from the lancet body.

The first convex part or concave part (see 303c in FIG.15B and 306 in FIG.16B) is formed in the protection cap. On the other hand, the fourth convex part or concave part is formed in the puncture device body (see 401c in FIG.15C and 401d in FIG.16C) . The lancet is attached to the puncture device body, so that the first convex part or concave part and the fourth convex part or concave part are engaged. By this engagement, the protection cap cannot move in the axial direction of the lancet body and cannot be removed from the puncture device body.

The puncture device also has a cutout part (see 405c in FIG.15C and 405d in FIG.16C) where the fourth convex part or concave part is not formed, in addition to the fourth convex part or concave part formed in the puncture device body. Further, a guide part (see 402c in FIG.15C and 402d in FIG.16C) is formed near the cutout part. When the protection cap of the lancet attached to the puncture device body is rotated predetermined degrees, the first convex part or concave part moves to the cutout part, and the first convex part or concave part and the fourth convex part or concave part are disengaged. When the protection cap is rotated predetermined degrees, the first convex part or concave part contacts with the guide part, and, when the protection cap is rotated further, the first convex part or concave part is guided by the guide part and pass through the cutout part, so that the protection cap is removed from the puncture device body.

The protection cap of the lancet attached to the puncture device body may be made rotatable with respect to the axis of the lancet body in both directions or in only one direction. The protection cap in the puncture device shown in FIG.15A and FIG.16A can be rotated in both directions. On the other hand in order to make the protection cap rotatable in only one direction, a member that restricts the rotation direction (see 403a in FIG.5B) may be provided in the fourth convex part or concave part. Further, the fifth convex part or concave part (see 401a in FIG.9) may be provided in puncture device body 101c or 101d. The fifth convex part or concave part engages with the first convex part or concave part of the protection cap if protection cap rotates in the inverse direction against the restriction.

The puncture device shown in FIG.15A has lancet 204c and puncture device body 101c, but is different from puncture device 101a shown in FIG.2 in that the puncture device does not have the puncture needle holder that holds the lancet. FIG.15B is a perspective view of lancet 204c. Lancet 204c has protection cap 202c and lancet body 203c. Similarly with protection cap 202a shown in FIG.4, the interior of protection cap 202c is made hollow, and claw 303c is formed in its inner periphery wall.

FIG.15C is a perspective view of puncture device body 101c. Flange 401c is formed in the tip part of puncture device body 101c, and cutout part 405c where flange 401c is lacking, is also provided. Guide 402c is placed in the position corresponding to cutout part 405c.

When lancet 204c is attached to puncture device body 101c, claw 303c of protection cap 202c which is integrated with lancet 204c, engages with flange 401c of puncture device body 101c. Further, when protection cap 202c is rotated with respect to the axis of lancet body 203c, claw 303c contacts with guide 402c. When protection cap 202c is rotated until claw 303c contacts with guide 402c, connecting part 302c is cut. When protection cap 202c is rotated further, claw 303c is guided by guide 402c and passes through cutout part 405c, so that protection cap 202c is removed from puncture device body 101c.

Next, FIG.16A shows the second example of the second puncture device. The puncture device shown in FIG.16A has lancet 204d and puncture device body 101d.

FIG.16B is a perspective view of lancet 204d, and lancet 204d has protection cap 202d and lancet body 203d. Lancet body 203d and protection cap 202d are connected separably via connecting part 302d. Lancet body 203d has grip part 207d to be held by plunger 103d of puncture device body 101d. On the other hand, angle guide 306 is formed in protection cap 202d. As described later, angle guide 306 is engaged with flange 401d formed in plunger 103d of the puncture device body.

FIG.16C is a perspective view of plunger 103d of puncture device body 101d. Plunger 103d holds grip part 207d of lancet body 203d. Further, flange 401d is formed in the tip part of plunger 103d, and cutout part 405d where flange 401d is lacking is also provided. Guide 402d is placed in the position corresponding to cutout part 405d.

When lancet 204d is attached to puncture device body 101d, flange 401d provided in plunger 103d inside puncture device body 101d is engaged with angle guide 306 of lancet 204d. Further, when protection cap 202d is rotated with respect to the axis of lancet body 203d, angle guide 306 contacts with guide 402d. When protection cap 202d is rotated until angle guide 306 contacts with guide 402d, connecting part 302d is cut. Further, when protection cap 202d is rotated, angle guide 306 is guided by guide 402d and passes through cutout part 405d, so that protection cap 202d is removed from puncture device body 101d.

### Industrial Applicability

The puncture needle cartridge and puncture device of the present invention enable the user to adequately remove the protection cap that protects the puncture needle, so that it is possible to perform puncturing safely without causing a needle-stick accident. Therefore, the puncture needle cartridge and puncture device of the present invention are suitable for use as a puncture device for collecting blood.

### Explanation of reference numerals

101a, 101c and 101d: puncture device body
102a, 102b, 102c and 102d: puncture needle cartridge
103a and 103d: plunger
201a, 201b, 201c and 201d: puncture needle holder
202a, 202b, 202c and 202d: protection cap
203a, 203b, 203c and 203d: lancet body
204a, 204b, 204c and 204d: lancet
205: inner diameter part
206a: sign
207a, 207b, 207c and 207d: grip part
301a, 301b, 301c and 301d: puncture needle
302a, 302b, 302c and 302d: connecting part
303a, 303b, 303c and 303d: claw
304a, 304b, 304c and 304d: inclination shape
305a and 305b: cross-shaped rib
306 angle guide
401a, 401b, 401c and 401d: flange
402a: slope
402b, 402c and 402d: guide
403a inverse rotation preventing rib
404a and 404b: cross-shaped groove
405a, 405b, 405c and 405d: cutout part

## Claims

1. A puncture needle cartridge comprising:
a lancet (204a,204b) including:
a lancet body (203a,203b);
a puncture needle (301a,301b);
a removable protection cap (202a,202b) covering the puncture needle (301a,301b) and including one of a first convex part and a first concave part (303a, 303b);
a connecting part (302a, 302b) that separably connects the lancet body (203a, 203b) and the removable protection cap (202a, 202b); and
a puncture needle holder (201a,201b) including:
one of a second convex part and a second concave part (401a,401b);
wherein the one of the first convex part and the first concave part (303a,303b) and the one of the second convex part and the second concave part (401a,401b) are engaged to restrict movement of the removable protection cap (202a,202b) in an axial direction of the lancet body (203a,203b); **characterized in that**
the one of the first convex part and the first concave part (303a,303b) is formed on an inside portion of the removable protection cap (202a,202b), the one of the second convex part and the second concave part (401a,401b) is formed on an outside portion of the puncture needle holder (201a,201b),
a cutout part (405a,405b) and a guide part (402a,402b) are formed on the outside portion of the puncture needle holder (201a,201b), and
the removable protection cap (202a,202b) is configured to rotate until the one of the first convex part and the first concave part (303a,303b) contacts with the guide part (402a, 402b) so that the one of the first convex part and the first concave part (303a,303b) pass through the cutout part (405a,405b), so that the removable protection cap (202a,202b) is removed from the puncture needle holder (201a,201b).

2. The puncture needle cartridge according to claim 1, wherein:
when the removable protection cap (202a,202b) rotates the predetermined degrees with respect to the axis of the lancet body (203a,203b), connecting part (302a,302b) is cut so that the lancet body (203a,203b) and the removable protection cap (202a,202b) are disconnected.

3. The puncture needle cartridge according to claim 1, wherein a contacting face between the guide part of the first convex part or concave part (303a,303b) and the guide part (402a) of the second convex part or concave part (401a,401b), has a sloping shape, a shape of a step or a shape of a semicircle, to prevent the guide part of the first convex part or concave part (303a,303b) from climbing over the guide part (402a) of the second convex part or concave part (401a,401b).

4. The puncture needle cartridge according to claim 1, further comprising a restriction part (403a) formed at the second convex part or concave part (401a,401b) of the puncture needle holder (201a,201b), wherein:
the restriction part (403a) restricts the rotation of the removable protection cap (202a,202b) in a direction other than a predetermined direction with respect to the axis of the lancet body (203a,203b).

5. The puncture needle cartridge according to claim 4, wherein a sign is provided to show the predetermined direction of rotation of the removable protection cap (202a,202b) with respect to the axis of the lancet body (203a,203b).

6. The puncture needle cartridge according to claim 4, further comprising a third convex part or concave part formed in the puncture needle holder (201a,201b), wherein:
if the removable protection cap (202a,202b) rotates in directions other than the predetermined direction of rotation, the third convex part or concave part (401a) is engaged with the first convex part (303a,303b) or concave part (306) of the removable protection cap (202a,202b) to restrict movement of the removable protection cap (202a,202b) in the axial direction.

7. The puncture needle cartridge according to claim 2 wherein the removable protection cap (202a,202b) can rotate the predetermined degrees in both rotation directions with respect to the axis of the lancet body (203a,203b).

8. A puncture device comprising:
the puncture needle cartridge according to claim 1; and
a puncture device body (101a) to which a puncture needle holder (201a,201b) is attached and which has a holding part that holds a grip part (207a,207b) of the lancet body (203a,203b).

## Patentansprüche

1. Stechnadel-Kassette, mit:
einer Lanzette (204a, 204b), die aufweist:
einen Lanzettenhauptteil (203a, 203b);
eine Stechnadel (301a, 301b);
eine abnehmbare Schutzkappe (200 2a, 200 2b), die die Stechnadel (301a, 301b) abdeckt und einen ersten konvexen Teil oder einen ersten konkaven Teil (303a, 303b) aufweist;
einen Verbindungsteil (302a, 302b), der separat den Lanzettenhauptteil (203a, 203b) und die abnehmbare Schutzkappe (202a, 202b) verbindet; und
einen Stechnadelhalter (201a, 201b), der aufweist:
einen zweiten konvexen Teil oder einen zweiten konkaven Teil (401a, 401b);
wobei der erste konvexe Teil oder der erste konkave Teil (303a, 303b) und der zweite konvexe Teil oder der zweite konkave Teil (401a, 401b) so im Eingriff sind, dass die Bewegung der abnehmbaren Schutzkappe (202a, 200 2b) in axialer Richtung des Lanzettenhauptteils (203a, 203b) begrenzt ist;
**dadurch gekennzeichnet, dass**
der erste konvexe Teil oder der erste konkave Teil (303a, 303b) auf einem Innenseitenbereich der abnehmbaren Schutzkappe (202a, 202b) ausgebildet ist, der zweite konvexe Teil oder der zweite konkave Teil (401a, 401b) auf einem Außenbereich des Stechnadelhalters (201a, 201b) ausgebildet ist, ein ausgeschnittener Teil (405a, 405b) und ein Führungsteil (402a, 402b) auf dem Außenseitenbereich des Stechnadelhalters (201a, 201b) ausgebildet sind, und
die abnehmbare Schutzkappe (202a, 202b) ausgebildet ist, sich zu drehen, bis der erste konvexe Teil oder der erste konkave Teil (303a, 303b) mit dem Führungsteil (402a, 402b) so in Kontakt tritt, dass der erste konvexe Teil oder der erste konkave Teil (303a, 303b) durch den ausgeschnittenen Teil (405a, 405b) so verläuft, dass die abnehmbare Schutzkappe (202a, 202b) von dem Stechnadelhalter (201a, 201b) abgenommen wird.

2. Stechnadel-Kassette nach Anspruch 1, wobei:
wenn die abnehmbare Schutzkappe (202a, 200 2b) sich um vorbestimmten Winkelgrade in Bezug auf die Achse des Lanzettenhauptteils (203a, 203b) dreht, der Verbindungsteil (302a, 302b) so geschnitten wird, dass der Lanzettenhauptteil (203a, 203b) und die abnehmbare Schutzkappe (202a, 202b) getrennt werden.

3. Stechnadel-Kassette nach Anspruch 1, wobei eine Kontaktfläche zwischen dem Führungsteil des ersten konvexen Teils oder konkaven Teils (303a, 303b) und dem Führungsteil (402a) des zweiten konvexen Teils oder konkaven Teils (401a, 401b) eine geneigte Form, eine Form einer Stufe oder eine Form eines Halbkreises hat, sodass verhindert wird, dass der Führungsteil des ersten konvexen Teils oder konkaven Teils (303a, 303b) sich über den Führungsteil (402a) des zweiten konvexen Teils oder konkaven Teils (401a, 401b) schiebt.

4. Stechnadel-Kassette nach Anspruch 1, die ferner einen Begrenzungsteil (403a) aufweist, der an dem zweiten konvexen Teil oder konkaven Teil (401a, 401b) des Stechnadelhalters (201a, 201b) ausgebildet ist, wobei:
der Begrenzungsteil (403a) die Drehung der abnehmbaren Schutzkappe (202a, 202b) in eine Richtung, die nicht eine vorbestimmte Dichtung in Bezug auf die Achse des Lanzettenhauptteils (203a, 203b) ist, begrenzt.

5. Stechnadel-Kassette nach Anspruch 4, wobei ein Zeichen vorgesehen ist, um die vorbestimmte Drehrichtung der abnehmbaren Schutzkappe (202a, 202b) in Bezug auf die Achse des Lanzettenhauptteils (203a, 203b) anzuzeigen.

6. Stechnadel-Kassette nach Anspruch 4, die ferner einen dritten konvexen Teil oder konkaven Teil aufweist, der in dem Stechnadelhalter (201a, 201b) ausgebildet ist, wobei:
wenn die abnehmbare Schutzkappe (202a, 202b) sich in Richtungen dreht, die nicht die vorbestimmte Drehrichtung sind, der dritte konvexe Teil oder konkave Teil (401a) mit dem ersten konvexen Teil (303a, 303b) oder konkaven Teil (306) der abnehmbaren Schutzkappe (202a, 202b) so in Eingriff tritt, dass eine Bewegung der abnehmbaren Schutzkappe (202a, 202b) in der axialen Richtung begrenzt ist.

7. Stechnadel-Kassette nach Anspruch 2, wobei die abnehmbare Schutzkappe (202a, 202b) um die vorbestimmten Winkelgrade in beiden Drehrichtungen in Bezug auf die Achse des Lanzettenhauptteils (203a, 203b) drehbar ist.

8. Stecheinrichtung, mit:
der Stechnadel-Kassette nach Anspruch 1; und
einem Stecheinrichtungshauptteil (101a), an welchem ein Stechnadelhalter (201a, 201b) angebracht ist und der einen Halteteil hat, der einen Griffteil (207a, 207b) des Lanzettenhauptteils (203a, 203b) hält.

## Revendications

1. Cartouche d'aiguille de ponction comprenant :
une lancette (204a, 204b) comprenant :
un corps de lancette (203a, 203b) ;
une aiguille de ponction (301a, 301b) ;
un capuchon de protection amovible (202a, 202b) recouvrant l'aiguille de ponction (301a, 301b) et comprenant l'une d'une première partie convexe et d'une première partie concave (303a, 303b) ;
une pièce de connexion (302a, 302b) qui relie séparément le corps de lancette (203a, 203b) et le capuchon de protection amovible (202a, 202b) ; et
un support d'aiguille de ponction (201a, 201b) comprenant :
l'une d'une seconde partie convexe et d'une seconde partie concave (401a, 401b) ;
l'une de la première partie convexe et de la première partie concave (303a, 303b) et l'une de la seconde partie convexe et de la seconde partie concave (401a, 401b) étant disposées pour restreindre le mouvement du capuchon de protection amovible (202a, 202b) dans un sens axial du corps de lancette (203a, 203b) ;
**caractérisée en ce que**
l'une de la première partie convexe et de la première partie concave (303a, 303b) est formée sur une partie intérieure du capuchon de protection amovible (202a, 202b),
l'une de la seconde partie convexe et de la seconde partie concave (401a, 401b) est formée sur une partie externe du support d'aiguille de ponction (201a, 201b),
une partie de découpe (405a, 405b) et une partie formant guide (402a, 402b) étant formées sur la partie externe du support d'aiguille de ponction (201a, 201b), et
le capuchon de protection amovible (202a, 202b) étant configuré pour tourner jusqu'à ce que l'une de la première partie convexe et de la première partie concave (303a, 303b) entre en contact avec la partie formant guide (402a, 402b) de sorte que l'une de la première partie convexe et de la première partie concave (303a, 303b) passe à travers la partie de découpe (405a, 405b), de sorte que le capuchon de protection amovible (202a, 202b) soit retiré du support d'aiguille de ponction (201a, 201b).

2. Cartouche d'aiguille de ponction selon la revendication 1, dans laquelle :
lorsque le capuchon de protection amovible (202a, 202b) fait tourner par degrés prédéterminés par rapport à l'axe du corps de lancette (203a, 203b), la pièce de liaison (302a, 302b) est coupée de sorte que le corps de lancette (203a, 203b) et le capuchon de protection amovible (202a, 202b) soient déconnectés.

3. Cartouche d'aiguille de ponction selon la revendication 1, dans laquelle une face de contact entre la partie formant guide de la première partie convexe ou de la partie concave (303a, 303b) et la partie formant guide (402a) de la seconde partie convexe ou partie concave (401a, 401b), présente une forme inclinée, une forme de marche ou une forme de demi-cercle, pour empêcher la partie formant guide de la première partie convexe ou partie concave (303a, 303b) de monter par-dessus la partie formant guide (402a) de la seconde partie convexe ou partie concave (401a, 401b).

4. Cartouche d'aiguille de ponction selon la revendication 1, comprenant en outre une partie de restriction (403a) formée au niveau de la seconde partie convexe ou partie concave (401a, 401b) du support d'aiguille de ponction (201a, 201b), dans laquelle :
la partie de restriction (403a) restreint la rotation du capuchon de protection amovible (202a, 202b) dans un sens autre qu'un sens prédéterminé par rapport à l'axe du corps de lancette (203a, 203b).

5. Cartouche d'aiguille de ponction selon la revendication 4, dans laquelle un signe est prévu pour montrer le sens de rotation prédéterminé du capuchon de protection amovible (202a, 202b) par rapport à l'axe du corps de lancette (203a, 203b).

6. Cartouche d'aiguille de ponction selon la revendication 4, comprenant en outre une troisième partie convexe ou partie concave formée dans le support d'aiguille de ponction (201a, 201b), dans laquelle :
si le capuchon de protection amovible (202a, 202b) tourne dans des sens autres que le sens de rotation prédéterminé, la troisième partie convexe ou partie concave (401a) est engagée avec la première partie convexe (303a, 303b) ou partie concave (306) du capuchon de protection amovible (202a, 202b) pour restreindre le mouvement du capuchon de protection amovible (202a, 202b) dans le sens axial.

7. Cartouche d'aiguille de ponction selon la revendication 2, le capuchon de protection amovible (202a, 202b) pouvant tourner selon les degrés prédéterminés dans les deux sens de rotation par rapport à l'axe du corps de lancette (203a, 203b).

8. Dispositif de ponction comprenant :
la cartouche d'aiguille de ponction selon la revendication 1 ; et
un corps de dispositif de ponction (101a) auquel un support d'aiguille de ponction (201a, 201b) est fixé et qui présente une pièce de maintien qui maintient une pièce de préhension (207a, 207b) du corps de lancette (203a, 203b) .
